# EUROPEAN PATENT SPECIFICATION

(11) **EP 2 427 204 B1**
(45) Date of publication and mention of the grant of the patent: **05.04.2017**
(21) Application number: 10772499.9
(22) Date of filing: 27.04.2010
(51) Int. Cl.: A61K 38/00, C07K 14/575

(54) **MELANOCORTIN RECEPTOR BINDING CONJUGATES**
MELANOCORTINREZEPTOR-BINDENDE KONJUGATE
CONJUGUÉS SE LIANT AU RÉCEPTEUR DE LA MÉLANOCORTINE

(30) Priority: 06.05.2009 US 175921 P; 07.05.2009 US 176294 P
(43) Date of publication of application: 14.03.2012
(73) Proprietor: Janssen Biotech, Inc., Horsham, PA 19044 (US)
(72) Inventor: HEAVNER, George, Radnor PA 19087 (US)
(74) Representative: Goodfellow, Hugh Robin
(86) International application number: PCT/US2010/032501
(87) International publication number: WO 2010/129248

(56) References cited:
- US-A1- 2007 015 257
- US-A1- 2008 031 848
- RUWE A R ET AL: "Semi-rigid tripeptide agonists of melanocortin receptors", BIOORGANIC & MEDICINAL CHEMISTRY LETTERS, PERGAMON, ELSEVIER SCIENCE, GB, vol. 19, no. 17, 1 September 2009 (2009-09-01), pages 5176-5181, XP026458585, ISSN: 0960-894X [retrieved on 2009-07-09]
- TODOROVIC A ET AL: "N-terminal fatty acylated His-DPhe-Arg-Trp-NH2 tetrapeptides: Influence of fatty acid chain length on potency and selectivity at the mouse melanocortin receptors and human melanocytes", JOURNAL OF MEDICINAL CHEMISTRY, AMERICAN CHEMICAL SOCIETY, US, vol. 48, no. 9, 30 March 2005 (2005-03-30) , pages 3328-3336, XP002375935, ISSN: 0022-2623, DOI: 10.1021/JM0490843

## Description

### Field of the Invention

The present invention relates to melanocortin receptor binding conjugates and methods of making and using the foregoing.

### Background of the Invention

Obesity is a chronic disease manifested by an excess of fat mass in proportion to body size. Today, every third American is considered overweight (Body Mass Index (BMI) >25 kg/m²). Obesity predisposes to Type 2 Diabetes, congestive heart failure, osteoarthritis, sleep apnea, Metabolic Syndrome, atherogenic dyslipidemia, elevated blood pressure and insulin resistance. Even a modest decrease in body weight (5-10% of initial body weight) may significantly lower the risk factors for developing obesity-associated diseases (Wing et al., Arch. Intern. Med. 147:1749-53 (1987); Tuomilehto et al., New Engl. J. Med. 344:1343-50 (2001); Knowler et al., New Engl. J Med. 346:393-403 (2002); Franz et al., Diabetes Care 25:148-98 (2002)). Additionally, treatment of obesity may be important from a mental health perspective due to the social stigma often attached to obese individuals in some cultures.

The melanocortin system plays a major role in the regulation of energy balance and food intake (Garfield et al., Trends Endocrinol Metab. 20:203-15 (2009)). In humans and rodents, loss of function mutations in the different components of the melanocortin system are closely correlated with obesity and related conditions. In mice, mutations within the melanocortin receptor 4 (MC4R), melanocortin receptor 3 (MC3R) or pro-opiomelanocortin (POMC) produce obesity, insulin resistance and hyperphagia (Goodfellow and Saunders, Curr. Topics Med. Chem. 3:855-83 (2003); Huszar et al., Cell 88:131-41 (1997); Yaswen et al., Nat. Med. 5:1066-70 (1999)). In man, mutations within POMC or MC4R lead to the development of obesity associated with increased food intake (Krude et al., Nat. Genet. 19:155-7 (1998); Yeo et al., Nature Genetics 20:111-2 (1998); Branson et al., New Engl. J. Med. 348:1096-103 (2003); Vaisse et al., J. Clin. Invest. 106:253-62 (2000); Ho and MacKenzie, J. Biol. Chem. 275:35816-22 (1999)). Pharmacological stimulation of MC4R leads to decreased food intake, increased energy expenditure and weight loss in rodents (Pierroz et al., Diabetes 51:1337-45 (2002)). In man, the intranasal administration of MC4R agonist alpha-MSH results in decreased body weight due to the decreased fat mass (Fehm et al., J. Clin. Endo. Metabol. 86:1144-48 (2001)). The wild type alpha-MSH is of limited use as a pharmaceutical due to its extremely short serum half-life. Alpha-MSH analogs have been described (Cai et al., Peptides 26:1481-5, (2005); Al-Obeidi t al., J. Am. Chem. Soc. 111:3413-3416 (1989); Kask et al., Endocrinology 139:5006-5014 (1998); Nijenhuis et al., Peptides 24:271-80, (2003); Schoth et al., Peptides 18:1009-13 (1997), Pat. Appl. No. WO06/073772; U.S. Pat. No. 6, 716, 810).

Alpha-MSH has potent anti-inflammatory effects mediated by centrally expressed melanocortin receptors. At the molecular level, alpha-MSH beneficially modulates various pathways implicated in regulation of inflammation and protection, such as NF-kB activation, production of proinflammatory cytokines and mediators, IL-10 synthesis, T cell and inflammatory cell proliferation and activity, inflammatory cell migration, expression of antioxidative enzymes, and apoptosis. The antiinflammatory effects of alpha-MSH have been validated in several inflammatory animal models ((for review see Brzoska et al., Endocr Rev. 29:581-602 (2008); Getting et al., ScientificWorldJournal. 9:1394-414 (2009), Maaser et al., Ann N Y Acad Sci. 1072:123-34 (2006)).

In recent years, the pro-opiomelanocortin (POMC)-derived peptides (melanocortins, e.g. alpha-MSH, beta-MSH and gamma-MSH, and adrenocorticotrophic hormone (ACTH)) having in common the tetrapeptide sequence His-Phe-Arg-Trp have been implicated in a plethora of diseases such as sexual impotence, frigidity, anorexia, cachexia, haemorrhagic shock, myocardial infarction, ischemia, neuropathic pain, rheumathoid arthritis, inflammatory bowel disease, nerve injury and neuropathies, by acting via different melanocortin receptors (for review Bertolini et al., Pharmacol Res. 59:13-47 (2009); Brzoska et al., Endocrine Rev. 29:581-602 (2008)).

Thus, a need exists to develop additional melanocortin receptor binding conjugates to treat obesity and other melanocortin system-mediated conditions.
US 2007/015257 describes alpha-MSH analog conjugates and their uses.

### Summary of the Invention

One aspect of the invention is an alpha-MSH derivative-Fc conjugate, wherein the alpha-MSH derivative has a structure shown in Formula X: 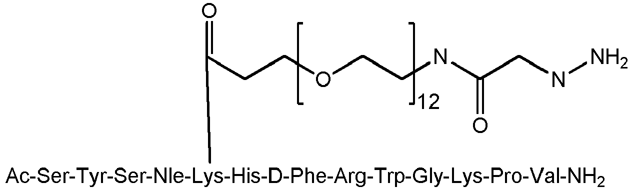 or Formula XI: 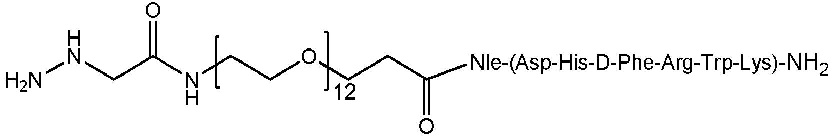 and the alpha-MSH derivative is conjugated to at least one Fc portion.
Also disclosed herein is an alpha-MSH derivative-Fc conjugate, wherein the conjugate has a structure shown in formula I:

B -(L)n - (X)m - (D)p - CH2 - CH3, (I),

wherein B is an alpha-MSH derivative;
L is a linker;
X is any naturally occurring amino acid.
D is is at least a portion of an immunoglobulin hinge region;
CH2 is at least a portion of an immunoglobulin CH2 constant domain;
CH3 is at least a portion of an immunoglobulin CH3 constant domain;
n is 0 or 1;
m is from 0 to 20; and
p is 0 or 1.

Another aspect of the invention is a pharmaceutical composition comprising the alpha-MSH derivative-Fc conjugate of the invention in combination with a pharmaceutically acceptable carrier.

Another aspect of the invention is a method of modulating a biological activity of a melanocortin receptor, comprising contacting the pharmaceutical composition of the invention with the melanocortin receptor.

Another aspect of the invention is an alpha-MSH derivative-Fc conjugate of the invention or pharmaceutical composition of the invention for use in treating a disease or condition.

### Detailed Description of the Invention

The term "alpha-MSH derivative" as used herein refers to a fragment, homolog, or analog of the "wild type alpha-MSH", and has at least one non-naturally occurring amino acid incorporated into the derivative. An alpha-MSH fragment is a peptide obtained after truncation of one or more amino acids from the N-terminus and/or C-terminus of the wild type alpha-MSH or an analog thereof. An alpha-MSH homolog is a peptide in which one or more amino acids have been added to the N-terminus and/or C-terminus of the wild type alpha-MSH, or fragments or analogs thereof. An alpha- MSH analog is a peptide in which one or more amino acids of the wild type alpha-MSH or fragment have been modified and/or substituted. An alpha-MSH derivative has sufficient homology to the wild type alpha-MSH as long as the analog at least partially retains the biological activity of the wild-type alpha-MSH. The alpha-MSH derivative may be a cyclic peptide, or have chemical modification of at least one amino acid side group, α-carbon atom, terminal amino group, or terminal carboxylic acid group. The "wild type alpha-MSH" is a 13 amino acid peptide processed from the precursor pro-opiomelanocortin (POMC), and binds with high affinity to the MC4R, and at lower affinity to the MC3R and MC5R. The "wild type alpha-MSH" has an amino acid sequence shown in SEQ ID NO: 1. Alpha-MSH derivatives can retain substantially the same, or a subset, of the biological activities of the wild type alpha-MSH.

"Melanocortin receptor" as used herein refers to the family of human melanocortin receptors comprising of melancortin 1 receptor (MC1R) (SEQ ID NO: 5), melanocortin 2 receptor (MC2R) (SEQ ID NO: 9), melanocortin 3 receptor (MC3R) (SEQ ID NO: 6), melanocortin 4 receptor (MC4R) (SEQ ID NO: 7), and melanocortin 5 receptor (MC5R) (SEQ ID NO: 8).

The terms "peptide" and "protein" are used interchangeably to refer to two or more amino acid residues linked together by amide bonds. The term is meant to include proteins, polypeptides, and peptides of any size, structure, or function. Typically, a peptide will be at least six amino acids long and a protein will be at least 50 amino acids long.

The term "conjugate" as used herein refers to the molecule formed as a result of covalent attachment of an alpha-MSH derivative to a Fc portion of an antibody. The alpha-MSH derivative can be coupled from the N-terminus or the C-terminus of the derivative to the C-terminus or N-terminus of the Fc portion, respectively, or from an internal amino acid. An optional linker may be inserted between the alpha-MSH derivative and the Fc portion. Exemplary covalent attachment of the alpha-MSH and the Fc can be done by a hydrazone or semicarbazone linkage.

The terms "Fc" or "Fc portion" as used herein refers to one of the well characterized fragments produced by digestion of an antibody with various peptidases, for example pepsin, and can include an antibody hinge region, and includes at least a portion of a hinge region, CH2, and CH3 domains.

The term "linker" (L) as used herein refers to an atom or a collection of atoms used to link alpha-MSH derivative and the Fc for example by one or more covalent bonds.

"Non-naturally occurring" or "unnatural" amino acid are used interchangeably and refer to amino acids not present in proteins isolated from nature. Exemplary non-naturally occurring amino acids are shown in Table 1, and also include D-amino acids, β-amino acids, pseudo-glutamate, γ-aminobutyrate, homocysteine, N-substituted amino acids (Simon et al., Proc. Natl. Acad. Sci. U.S.A. 89:9367-71 (1992); WO91/19735, U.S. Pat. No. 5,646,285), α-aminomethyleneoxy acetic acids (an amino acid-Gly dipeptide isostere), and α-aminooxy acids and other amino acid derivatives having non-genetically non-encoded side chain function groups.

The term "ethylene glycol unit" as used herein refers to linear oligomers composed of repeating ethyleneoxy units. The termini of the ethyleneoxy units can be functionalized by the addition of amino and carboxylic acid groups and derivatives thereof. An exemplary amino group is a hydrazine.

"Increase in serum half-life" or "increased t_{1/2} " as used herein refers to the positive change in circulating half-life of a modified biologically active molecule relative to its non-modified form. Serum half-life of proteins can be determined in humans or in animal models, such as *Macaca fascicularis* or *Papio cynocephalus* (cynomolgus monkey). An exemplary incrase in serum half-life is about two-fold, but a smaller increase may be useful.

The present invention relates to alpha-MSH derivative-Fc conjugates that incorporate non-naturally occurring amino acids or additional organic moieties in the alpha-MSH derivative, that can result in improved properties in the alpha-MSH derivative-Fc conjugates.

**Table 1.**

| Non-natural amino acid | Symbol |
|---|---|
| 2-Aminoadipic acid | Aad |
| beta-Alanine, beta-Aminopropionic acid | bAla |
| 2-Aminobutyric acid | Abu |
| 4-Aminobutyric acid, piperidinic acid | 4Abu |
| 6-Aminocaproic acid | Acp |
| 2-Aminoheptanoic acid | Ahe |
| 2-Aminoisobutyric acid | Aib |
| 2-Aminopimelic acid | Apm |
| 2,4-Diaminobutyric acid | Dbu |
| Desmosine | Des |
| 2,2' -Diaminopimelic acid | Dpm |
| 2,3-Diaminopropionic acid | Dpr |
| N-Ethylglycine | EtGly |
| N-Ethylasparagine | EtAsn |
| Hydroxylysine | Hyl |
| allo-Hydroxylysine | AHyl |
| 3-Hydroxyproline | 3Hyp |
| 4-Hydroxyproline | 4Hyp |
| Isodesmosine | Ide |
| allo-Isoleucine | Alle |
| N-Methylglycine, sarcosine | MeGly |
| N-Methylisoleucine | Melle |
| 6-N-Methyllysine | MeLys |
| N-Methylvaline | MeVal |
| Norvaline | Nva |
| Norleucine | Nle |
| Ornithine | Orn |

Also disclosed herein is an alpha-MSH-Fc conjugate, wherein the conjugate has a structure shown in formula I:

B -(L)n - (X)m - (D)p - CH2 - CH3, (I),

wherein B is an alpha-MSH,
L is a linker,
X is a naturally occurring amino acid,
D is is at least a portion of an immunoglobulin hinge region,
CH2 is at least a portion of an immunoglobulin CH2 constant domain,
CH3 is at least a portion of an immunoglobulin CH3 constant domain,
n is 0 or 1;
m is from 0 to 20; and
p is 0 or 1.

In some instances the alpha-MSH derivative has a structure shown in Formula II:

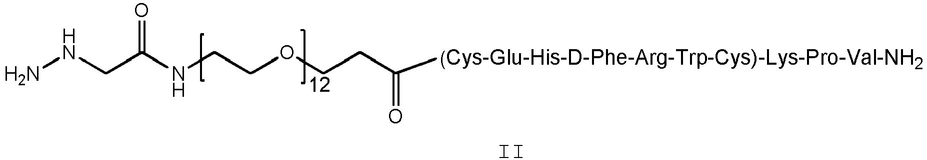

In some instances the alpha-MSH derivative has a structure shown in Formula III:

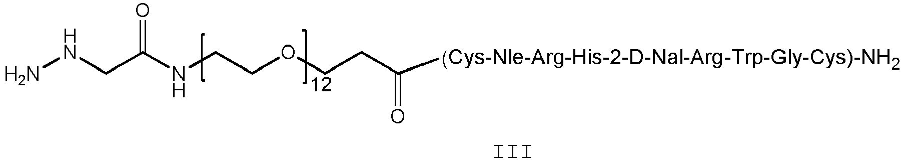

In some instances the alpha-MSH derivative has a structure shown in Formula IV:

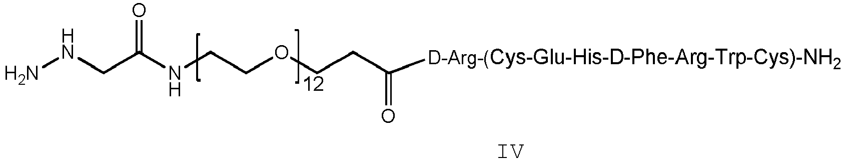

In some instances the alpha-MSH derivative has a structure shown in Formula V:

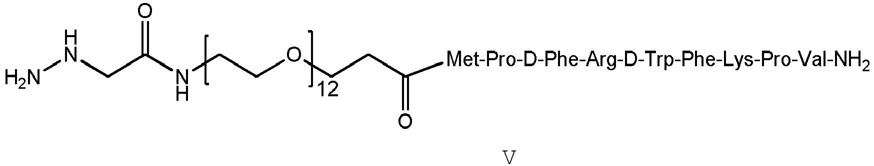

In some instances the alpha-MSH derivative has a structure shown in Formula VI:

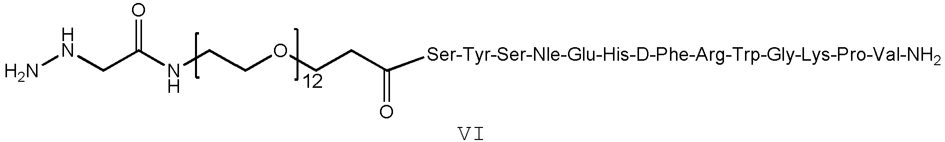

In some instances the alpha-MSH derivative has a structure shown in Formula VII:

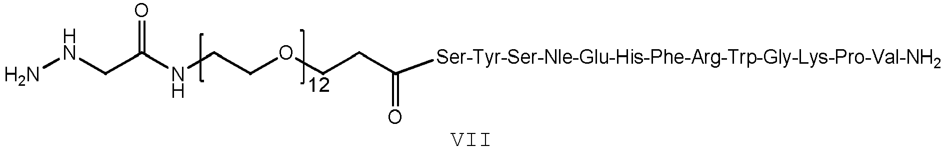

In some instances the alpha-MSH derivative has a structure shown in Formula VIII:

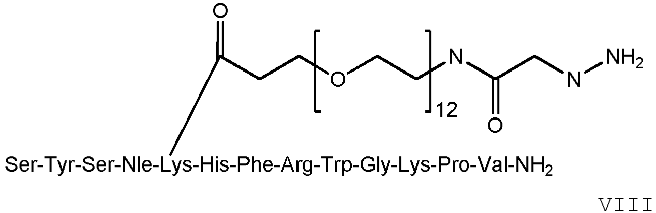

In some instances the alpha-MSH derivative has a structure shown in Formula IX:

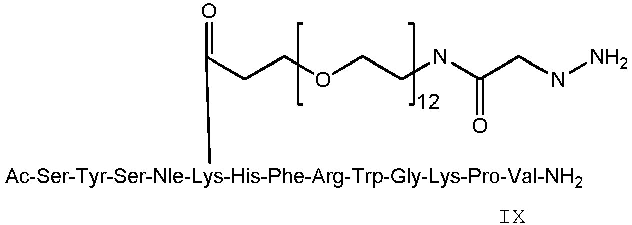

In one embodiment of the invention, the alpha-MSH derivative has a structure shown in Formula X:

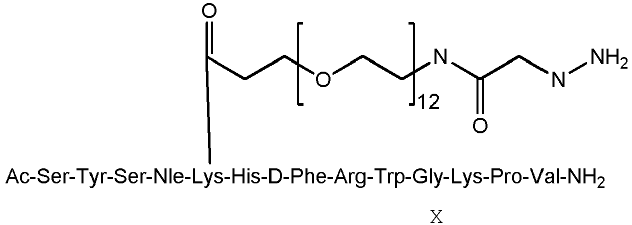

In other embodiments of the invention, the alpha-MSH derivative has a structure shown in Formula XI:

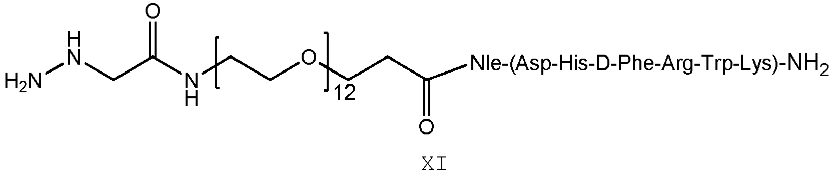

In some instances the alpha-MSH derivative has a structure shown in Formula XII:

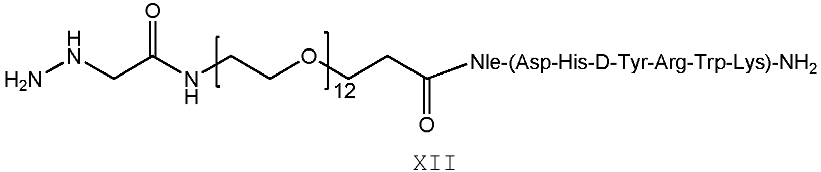

In some instances the alpha-MSH derivative has a structure shown in Formula XIII:

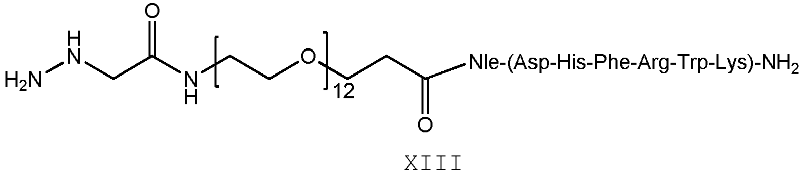

In some instances the alpha-MSH derivative has a structure shown in Formula XIV:

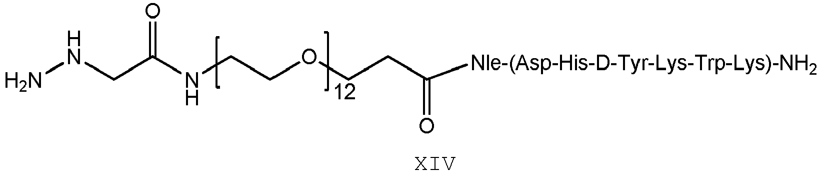

In some instances the alpha-MSH derivative has a structure shown in Formula XV:

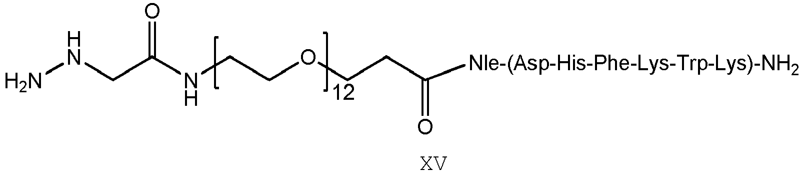

The alpha-MSH derivative can be produced by for example chemical synthesis or recombinant expression or a combination thereof, using well known methods. For solid phase peptide synthesis, t-Boc (tert-Butyloxy carbonyl) and Fmoc (Fluorenyl-methoxy-carbonyl) chemistry, referring to the N-terminal protecting groups, on polyamide or polystyrene resin can be used (Merrifield, Ann N Y Acad Sci. 104:161-71 (1963)).

For recombinant production, non-naturally occuring amino acid residues can be introduced using for example codon suppression to introduce an aldehyde or ketone functional group in any suitable position within alpha-MSH derivative or by expressing the alpha-MSH derivative in the Pseudomonas host cells (Pat. Appl. No. WO06/132969). Exemplary non-naturally occurring amino acids are discussed above.

Amino acids in a polypeptide that are essential for function can be identified by well known methods, for example generating variants using chemical synthesis or using site-directed mutagenesis, and testing the variants for desired biological activity, such as the ability to induce signaling downstream through a cognate receptor.

The alpha-MSH derivative can further be modified by covalent attachment of at least one organic moiety to, for example, improve pharmacokinetic properties of the molecule. The organic moiety can be, for example, fatty acid, fatty acid ester, polyalkane glycol including polyethylene glycol (PEG), carbohydrate polymer, amino acid polymer or polyvinyl pyrolidone. The fatty acid and the fatty acid ester can comprise from about 8-40 carbon atoms. Exemplary fatty acids are laurate, myristate, stearate, arachidate, behenate, n-triacontanoate , n-tetracontanoate, oleate, arachidonate, octanedioic acid, tetradecanedioic acid, octadecanedioic acid, and docosanedioic acid. The polyalkane glycol can have a molecular weight of about 800 to about 120,000 Daltons. The polyethyleneglygol (PEG) can have a molecular weight of between about 100-5000 kD, or between about 100-500 kD. The PEG, fatty acids and their esters can be derivatized with terminal amino, hydroxyl, mercapto, and/or carboxy groups. The organic moiety can be coupled to the alpha-MSH derivative using well know methods (Hermanson, Bioconjugate Techniques, Academic Press: San Diego, CA (1996); Kolb, Finn and Sharpless, "Click Chemistry: Diverse Chemical Function from a Few Good Reactions". Angewandte Chemie International Edition 40:2004-21 (2001); Evans, Australian J. Chem. 60:384-95 (2007)). Exemplary methods for selective attachment of PEG have been described (Pat. Appl. No. WO99/45026, Pat. Appl. No. WO 99/03887, U.S. Pat. No. 5,206,344 and U.S. Pat. No. 5,766,897).

The Fc portion of the alpha-MSH derivative-Fc conjugates of the present invention can be derived from an intact naturally occurring isolated antibody after for example papain cleavage, or can be synthesized *de novo* recombinantly or chemically using standard methods. The sequences of human and animal immunoglobulins can be found at the ImMunoGeneTics database (http://_www._imgt_org) or at Kabat, et al. Sequences of Proteins of Immunological Interest, U.S. Dept. Health (1983). Sequences derived from human germline information as well as known and useful therapeutic human antibody sequences and variations thereof have been summarized in Pat. Appl. No. WO05/005604. The Fc portion may be derived from any immunoglobulin class, e.g. IgA₁, IgA₂, IgD, IgE, IgG₁, IgG2₁, IgG3₁, IgG2₄, and IgM heavy chains. Exemplary Fc portions have amino acid sequences shown in SEQ ID NOs: 2-3. The Fc portion may be produced in a glycosylated or non-glycosylated form by expressing the Fc in mammalian or prokaryotic cells, respectively. The glycosylated antibodies are more resistant to papain digestion than the deglycosylated or aglycosylated or non-glycosylated antibodies, thus the deglycosylation step should be performed following papain digestion when producing Fc portions from naturally occurring isolated antibodies.

The Fc domain mediates antibody effector functions such as complement binding and antibody-dependent cell cytoxicity (ADCC). Certain natural and synthetic variants of the Fc-region polypeptides sequences with altered effector functions include mutant polypeptides as described in, for example, U.S. Pat No. 5,624,821, U.S. Pat No. 6,528,624, U.S. Pat No. 7,122,637, U.S. Pat No. 7,183,387. Antibody effector functions can also be dependent on the presence of the glycans attached to the Fc in the CH2 domain (Jefferis & Lund, Immunol. Letters. 82:57-65, (2002)). The non-glycosylated Fc domains incapable of inducing effector functions can be generated by expressing the Fc domains recombinantly in bacterial host cell, or the glycans can be removed enzymatically using glycosidases.

The alpha-MSH derivative may be conjugated to at least one Fc portion in an immunoglobulin, optionally via the linker. Non-identical alpha-MSH derivatives can be conjugated to the Fc portion when more than one alpha-MSH derivative is conjugated. Conjugation can be done from any residue on the alpha-MSH derivative so long as the final alpha-MSH derivative-Fc conjugate displays the desired biological activity. Biological activity may be measured by *in vitro* assays, for example binding activity, by *in vivo* activity such as in animal models of disease, or by the response of a subject following administration of the conjugate. Exemplary biological activity measures increase in intracellular cAMP upon MC4R stimulation as described in the Examples.

The conjugation of the alpha-MSH derivative to the Fc portion can be done for example by reductive alkylation between a nucleophilic group that has been introduced into the alpha-MSH derivative and a reactive carbonyl in the Fc. Exemplary nucleophilic groups are a primary amine, hydrazine, acyl hydrazide, carbazide, semicarbazide or thiocarbazide groups. Exemplary carbonyl groups are an aldehyde or ketone groups. The nucleophilic group can be attached to the N-terminus of the derivative, the C-terminus of the derivative or any side chain containing an amino, hydroxyl, thiol, carboxylic acid or carboxamide functionality, to a similar group on a non-natural amino acid, or to an organic moiety attached to the alpha-MSH derivative. The activation of the alpha-MSH derivative for conjugation to the Fc-domain can be done during the solid phase synthesis using tri-Boc-hydrazinoacetic acid at the final residue prior to cleavage from the resin to give the hydrazine derivatized alpha-MSH derivative.

The N-terminal residue of the Fc portion is prepared for conjugation by creating a reactive (electrophilic) carbonyl from for example aldehydes or ketones. Fc portions containing an N-terminal threonine (Thr) or serine (Ser), e.g. a reactive carbonyl, can be oxidized with periodic acid to give N-terminal glycoxylic acid derivatives (Geoghegan and Stroh, Bioconjugate Chem. 3:138-46 (1992); U.S. Pat. No. 5,362,852; Garnter et al., Bioconjugate Chem. 7:38-44, (1996); Pat. Appl. No. WO98/05363; U.S. Pat. Appl. No. US09/0181037). The Fc portion may naturally have a serine or threonine or may be engineered or chemically altered to display a serine or threonine at the N-terminus by well known methods, for example by synthesis of glyoxylyl peptides using an Fmoc-protected α, α'-diaminoacetic acid derivative ((Fmoc-NH)₂CHCO₂H) (Far and Melnyk, J. Peptide Sci. 11:424-30, 2005). Alternatively, the N-terminal Ser or Thr residue can be generated in proteins by utilizing reverse proteolysis using trypsin or carboxypeptidase Y (Rose et al., Biochem J. 211:671-6 (1983)), or the glyoxyl transamination reaction can be used to generate an aldehyde ((Dixon and Fields, Methods Enzymol. 25:409-19 (1979), US Pat. No. 6,077,393).

If the Fc contains N- or O-linked glycosyl groups, the carbohydrate is susceptible to oxidation by oxidizing agents used to affect the formation of the N-terminal glyoxal group, producing additional reactive carbonyl species. Carbohydrates can be removed prior to chemical linking using PNGase followed by purification by hydrophobic interaction HPLC.

The stochiometry of incorporation can be controlled by adjusting the stochiometry of the reaction or by incorporating and controlling suitable steric interactions.

An exemplary alpha-MSH derivative-Fc conjugate is formed by oxidizing the N-terminal Thr of IgG₁ Fc to form glyoxylate, conjugating the oxidized Fc with the alpha-MSH derivative of Formula IX via a hydrazine bond in the attached organic moiety (PEG), followed by NaBH₃CN reduction to stabilize the conjugate.

The alpha-MSH derivative may be conjugated to the Fc via a linker. Exemplary linkers include alkyl linkers such as -NH-(CH₂)_{S}-C(O)-, wherein s=2-20. The alkyl linkers may further be substituted by any non-sterically hindering group such as lower alkyl (e.g., C₁ - C₆) lower acyl, halogen (e.g., Cl, Br), CN, NH₂, or phenyl. Exemplary polymer linkers are polyethylene glycol (PEG), polyvinyl pyrrolidone, polyvinyl alcohol, polyamino acids, divinylether maleic anhydride, N-(2-Hydroxypropyl)-methacrylamide, dextran, dextran derivatives including dextran sulfate, polypropylene glycol, polyoxyethylated polyol, heparin, heparin fragments, polysaccharides, cellulose and cellulose derivatives, including methylcellulose and carboxymethyl cellulose, starch and starch derivatives, polyalkylene glycol and derivatives thereof, copolymers of polyalkylene glycols and derivatives thereof, polyvinyl ethyl ethers, and α,β-Poly[(2-hydroxyethyl)-DL-aspartamide, and the like, or mixtures thereof. The polyethyleneglycol (PEG) linker can have the same properties as described above for the PEG modification of the alpha-MSH.

The linker may include polymer chains or units that are biostable or biodegradable. For example, polymers with repeat linkages may have varying degrees of stability under physiological conditions depending on bond lability. Polymers with such bonds can be categorized by their relative rates of hydrolysis under physiological conditions based on known hydrolysis rates of low molecular weight analogs, e.g., from less stable to more stable polycarbonates (-O-C(O)-O-)> polyesters (-C(O)-O-) > polyurethanes (-NH-C(O)-O-)> polyorthoesters (-O-C((OR)(R'))-O-)>polyamides (-C(O)-NH-). Similarly, the linkage systems attaching a watersoluble polymer to a target molecule may be biostable or biodegradable, for example, from less stable to more stable carbonate (-O-C(O)-O-)>ester (-C(O)-O-)> urethane (-NH-C(O)-O-)>orthoester (-O-C((OR)(R'))-O-)>amide (-C(O)-NH-).

The linker can be added to the alpha-MSH derivative during solid phase synthesis, by coupling the linker having protected bifunctional groups for example to the deprotected N-terminal amino group or any other amino acid having side chain amino group (for example, lysine, diamino butyric acid, and 4-amino phenylalanine) of the alpha-MSH derivative. The linker can be conjugated at the C-terminus of the alpha-MSH derivative by direct coupling of the linker to the resin, followed by assembly of the peptide and cleavage of the alpha-MSH derivative -linker from the resin by hydrazine or a hydrazine derivative. The peptide may also be prepared on a resin such as the Universal PEG NovaTag resin (Novabiochem). The methods of coupling are well known.

The alpha-MSH derivative-Fc conjugate may include one or more naturally occurring amino acids (X) between the linker (L) and the hinge (D), as shown in Formula I. The amino acids between the linker and the hinge are typically derived from a variable region of an antibody, and are inserted to increase the distance between the alpha-MSH derivative and the Fc, thereby faclilitating proper conformational structure and activity of the alpha-MSH derivatives. Exemplary naturally occurring amino acids in the alpha-MSH derivative-Fc conjugate can be a peptide having a sequence GTLVTVSS (SEQ ID NO: 4). The sequence may be derivatized to allow covalent bond formation with the linker.

### Methods of Treatment

The alpha-MSH derivative-Fc conjugates of the present invention can be used to treat a wide variety of diseases and conditions. While not wishing to be bound by any particular theory, the alpha-MSH-Fc conjugates of the invention may stimulate the melanocortin system by agonizing the MC3R and/or the MC4R. The methods disclosed herein may be used to treat a subject belonging to any classification. Examples of such subjects include mammals such as humans, rodents, dogs, cats and farm animals. For example, the alpha-MSH-Fc conjugates of the invention are useful in agonizing melanocortin receptors, in the treatment of obesity, type II diabetes, and metabolic syndrome, and are also useful in the preparation of a medicament for such treatment wherein the medicament is prepared for administration in dosages defined herein.

Disease and conditions that may be treated by administration of the alpha-MSH derivative-Fc conjugates of the present invention include type I diabetes, type II diabetes, stroke (Pat. Appl. No. WO00/16797), myocardial infarction (Pat. Appl. No. WO98/08531), obesity (Pat. Appl. No. WO98/19698), catabolic changes after surgery (U.S. Pat. No. 6,006,753), functional dyspepsia and irritable bowel syndrome (Pat. Appl. No. WO99/64060). Subjects with impaired glucose tolerance, impaired fasting glucose, overweight subjects with body weight index (BMI) over 25, subjects with a partial pancreatectomy or gestational diabetes, and subjects who have had acute or chronic pancreatitis may be treated with alpha-MSH derivative-Fc conjugates of the present invention. Also included are subjects requiring prophylactic treatment with an MSH compound, for example, subjects at risk for developing non-insulin dependent diabetes (Pat. Appl. No. WO00/07617).

Specific biological effects can be elicited by treatment with an alpha-MSH derivative of limited function. Treatment of a subject with an alpha-MSH derivative having a subset of the biological activities of the wild type alpha-MSH can have fewer side effects in a subject relative to treatment with the naturally occurring form of the protein.

### Administration/ pharmaceutical compositions

The "therapeutically effective amount" of the alpha-MSH derivative-Fc conjugate in the treatment or prevention of conditions where modulation of the melanocortin system is desirable can be determined by standard research techniques. For example, the dosage of the agent that will be effective in the treatment or prevention of obesity, diabetes or insulin resistance can be determined by administering the agent to relevant animal models.

In addition, *in vitro* assays can optionally be employed to help identify optimal dosage ranges. Selection of a particular effective dose can be determined (for example, via clinical trials) by those skilled in the art based upon the consideration of several factors. Such factors include the disease to be treated or prevented, the symptoms involved, the patient's body mass, the patient's fasting glucose and insulin and other factors known by the skilled artisan. The precise dose to be employed in the formulation will also depend on the route of administration, and the severity of disease, and should be decided according to the judgment of the practitioner and each patient's circumstances. Effective doses can be extrapolated from dose-response curves derived from *in vitro* or animal model test systems.

In the methods disclosed herein, the alpha-MSH-Fc conjugates may be administered singly or in combination with at least one other molecule. Such additional molecules may be other melanocortin receptor agonists or molecules with a therapeutic benefit not mediated by melanocortin receptor signaling. Small molecules that reduce body weight or improve insulin resistance are examples of such additional molecules.

The mode of administration for therapeutic use of the alpha-MSH derivative-Fc conjugates of the invention may be any suitable route that delivers the alpha-MSH derivative-Fc conjugates to the host. Pharmaceutical compositions of the alpha-MSH derivative-Fc conjugates can be administrated using for example oral, rectal, nasal, lower respiratory, intramuscular, intravenous or subcutaneous routes.

The alpha-MSH derivative-Fc conjugates of the invention may be prepared as pharmaceutical compositions containing an effective amount of the alpha-MSH derivative-Fc conjugate as an active ingredient in a pharmaceutically acceptable carrier. The term "carrier" refers to a diluent, adjuvant, excipient, or vehicle with which the active compound is administered. Such pharmaceutical vehicles can be liquids, such as water and oils, including those of petroleum, animal, vegetable or synthetic origin, such as peanut oil, soybean oil, mineral oil, sesame oil and the like. For example, 0.4% saline and 0.3% glycine can be used. These solutions are sterile and generally free of particulate matter. They may be sterilized by conventional, well-known sterilization techniques (e.g., filtration). The compositions may contain pharmaceutically acceptable auxiliary substances as required to approximate physiological conditions such as pH adjusting and buffering agents, stabilizing, thickening, lubricating and coloring agents, etc. The concentration of the alpha-MSH derivative-Fc conjugates of the invention in such pharmaceutical formulation can vary widely, i.e., from less than about 0.5%, usually at or at least about 1% to as much as 15 or 20% by weight and will be selected primarily based on required dose, fluid volumes, viscosities, etc., according to the particular mode of administration selected.

Thus, a pharmaceutical composition of the invention for intramuscular injection could be prepared to contain 1 ml sterile buffered water, and between about 1 ng to about 100 mg, e.g. about 50 ng to about 30 mg or more preferably, about 5 mg to about 25 mg, of the alpha-MSH derivative-Fc conjugate of the invention. Similarly, a pharmaceutical composition of the invention for intravenous infusion could be made up to contain about 250 ml of sterile Ringer's solution, and about 1 mg to about 30 mg and preferably 5 mg to about 25 mg of the alpha-MSH derivative-Fc of the invention. Actual methods for preparing parenterally administrable compositions are well known and are described in more detail in, for example, "Remington's Pharmaceutical Science", 15th ed., Mack Publishing Company, Easton, PA.

The alpha-MSH derivative-Fc conjugates of the invention can be lyophilized for storage and reconstituted in a suitable carrier prior to use. This technique has been shown to be effective with other protein preparations and art-known lyophilization and reconstitution techniques can be employed.

While having described the invention in general terms, the embodiments of the invention will be further disclosed in the following examples.

### Example I

### Synthesis of alpha-MSH derivatives

The peptides were prepared on an ABI 433A Peptide Synthesizer using SynthAssist 2.0 Version for Fmoc/HBTU chemistry by the Fastmoc 0.1 mM Monitoring Previous Peak software.

### Peptides of Formulae II-IV.

NovaSyn TGR Novabiochem amide resin (0.1 mmol) was used in the synthesis. The final two amino acids derivatives in the synthesis were tri-BOC-hydrazinoacetic acid and O-(N-Fmoc-2-aminoethyl)-O'-(2-carboxyethyl)-undecaethyleneglycol.

After synthesis, the resin was washed 3 x 2 min with N-methylpyrrolidone, 1 x 2 min with methylene chloride/N-methylpyrrolidone, 3 x 2 min with methylene chloride, 3 x 2 min with methanol, 1 x 2 min with ethyl ether and dried under reduced pressure for two hours.

The peptides were cleaved from the resin by stirring in a scintillation vial using 20 mL of a cleavage mixture of trifluoroacetic acid (30 mL), phenol (2.25 g), dithiothrietol (1.5 g), thioanisole (1.5 mL), triisopropylsilane (1.5 mL), and water (1.5 mL) for four hours at ambient temperature. The resin was removed by filtration and the peptide was precipitated by the addition of precooled ethyl ether (600 mL). The resulting solid was isolated by filtration and washed with ethyl ether and dried under reduced pressure. For synthesis of some peptides, 15 ml of cleavage mixture was used and the peptides precipitated with 400 mL precooled ethyl ether.

### Peptide cyclization

The crude material from the cleavage was dissolved in 50 ml of water with a trace of acetic acid. 600 ml of water was adjusted to pH 8.0 with 1 M NH₄OH. A solution of 0.01M K₃Fe(CN)₆ was prepared in water. Over the course of 4 hours, aliquots of the peptide were added to the water and the pH adjusted to 8.0 with 1 M NH₄OH. Aliquots of the ferricyanide solution were added to maintain a light yellow color. After the addition, the solution was stirred for an additional hour. To the solution was added 50 ml of BioRad AG-4-X3, 100-200 mesh, free base form. The solution was stirred for 1 hour and the resin removed by filtration. The filtrate was lyophilized to give the crude cyclic peptide.

The crude peptide was purified on two Vydac C-18 columns (10 mm, 2.5 x 25 cm), using a gradient of 0-100%, 10-90% or 10-80%, depending on a peptide (Buffer A = 0.1% trifluoroacetic acid in water, Buffer B = 0.1% trifluoroacetic acid in 80% acetonitrile/20% water (GAH) in water) over 60 min at a flow rate of 6 mL/min. Fractions were collected, analyzed by HPLC and the pure fractions pooled and lyophilized.

Peptides of Formulae V-VII. Synthesis and purification was done as describe for Formulae II-IV peptides, except that the cyclization step was omitted.

Peptides of Formulae VIII and IX. 0.25 mmol NovaSyn TGR Novabiochem amide resin was used in the synthesis. For Lys⁵, DDE-Lys(FMOC) was used. After the coupling of DDE-Lys(FMOC), tri-BOC-hydrazinoacetic acid and O-(N-Fmoc-2-aminoethyl)-O'-(2-carboxyethyl)-undecaethyleneglycol were added. The DDE group was removed by treating the resin in a manual shaker 4 x 2 minutes with a solution of 2% anhydrous hydrazine in DMF. The resin was returned to the synthesizer and the final four amino acids were coupled and the N-terminal FMOC group removed.

The peptides were cleaved from the resin by stirring in a scintillation vial using 15 ml of a mixture of 20 ml of trifluoroacetic acid, 3 g of phenol, 6 ml of ethanedithiol, 1 ml of thioanisole and 1 ml of water for 4 hr at ambient temperature. The resin was removed by filtration and the peptide was precipitated by the addition of precooled ethyl ether (200 mL), and the peptides were purified as described above.

Peptide of Formula X. 0.50 g of 0.20 meq/g NovaSyn TGR Novabiochem amide resin was used. The protecting groups used were N-terminal FMOC, His(Trt), Arg(Pmc), Ser(t-Bu), Lys(BOC) and Lys(DDE). Deprotection was with 5% DBU in piperidine, diluted according to the FastMoc program. The residues were coupled to give Ser(O-t-Bu)-Tyr(BOC)-Ser(O-t-Bu)-Nle-Lys(DDE)-His(Trt)-Phe-Arg(Pmc)-Trp(BOC)-Gly-Lys(BOC)-Pro-Val-Resin. The resin was acetylated manually by treatment for 10 minutes with a solution of 1 ml acetic anhydride, 9 ml DMF and 0.1 ml DIEA. After washing with DMF, the DDE group was removed by treating the resin in a manual shaker 4 x 2 minutes with a solution of 2% anhydrous hydrazine in DMF. The resin was returned to the synthesizer and the final amino acids were coupled according to the above protocol.

The peptide resin (0.748 g) was simultaneously deprotected and cleaved from the resin with 10 ml of a mixture of TFA/ phenol/ ethanedithiol/ thioanisole/ water 10ml/1.5 g/3 ml/0.5 ml/0.5 ml for 4 hr at ambient temperature. The resin was removed by filtration and the filtrate added to 200 ml of diethyl ether and stirred for ½ hr. The resulting solid was isolated by centrifugation, washed well with ether and dried under reduced pressure to give 0.26 g of a white solid.

The crude material was injected in three equal aliquots onto two Vydac C-18 (25 x 250 mm, 10µ)columns in tandem and eluted using a 20 - 100% (80% acetonitrile/0.1% trifluoroacetic acid in water) over 120 min at a flow rate of 5 mL/min. Yields of the peptides were typically between 36-65 mg. The observed and calculated molecular weights were in good accordance. The characteristics of the synthesized alpha-MSH derivatives are shown in Table 2.

**Table 2.**

| Formula | Molecular formula | MW (Da) observed |
|---|---|---|
| II | C₈₈H₁₄1N₂₁O₂₆S₂ | 1,973.0 |
| III | C₈₅H₁₃4N₂₂O₂₃S₂ | 1,896.9 |
| IV | C₇₈H₁₂₅N₂₁O₂₄S₂ | 1,805.8 |
| V | C₉₀H₁₄₄N₁₈O₂₃S | 1,878.9 |
| VI | C₁₀5H₁₆₆N₂₄O₃₂ | 2,277.0 |
| VII | C₁₀₅H₁₆₆N₂₄O₃₂ | 2,277.0 |
| VIII | C₁₀₆H₁₇₁N₂₅O₃₀ | 2,275.6 |
| IX | C₁₀₈H₁₇₃N₂₅O₃₁ | 2,318.2 |
| X | C₁₃₅H₂₂₆N₂₆O₄₄ | 2,917.7 |
| XI | C₇₇H₁₂4N₁₈O₂₂ | 1,654.5 |
| XII | C₇₇H₁₂4N₁₈O₂₃ | 1,670.6 |
| XIII | C₇₇H₁₂₄N₁₈O₂₂ | 1,654.7 |
| XIV | C₇₇H₁₂₄N₁₈O₂₃ | 1,670.6 |
| XV | C₇₇H₁₂₄N₁₈O₂₂ | 1,654.5 |

Peptide of Formulae XI-XV. 1.25 g of 0.20 meq/g NovaSyn TGR Novabiochem amide resin was used. The protecting groups used were N-terminal FMOC, His(Trt), Arg(Pmc), Tyr(BOC), Lys(BOC), Lys(Mtt) and Asp(O-Dmb). Deprotection was with piperidine, according to the FastMoc program. The residues were coupled to give,for example, FMOC-Asp(O-Dmb)-His(Trt),Phe-Lys(BOC)-Tyr(BOC)-Lys(Mtt)-Resin. The resin was treated manually with 1% TFA in methylene chloride for 20 minutes and then washed well with methylene chloride and NMP. The partially deprotected resis peptide was treated with HATU in a mixture of DMF and NMP for 30 min at ambient temperature to form the lactam. Folowing formation of the lactam the resin was returned to the synthesizer for the final couplings of FMOC-Nle, FMOC-PEG₁₂-CO₂H and BOC₃-hydrazinoacetic acid. The peptide resin (2.15 g) was simultaneously deprotected and cleaved from the resin with 10 ml of a mixture of TFA/ phenol/ ethanedithiol/ thioanisole/ water 10ml/1.5 g/3 ml/0.5 ml/0.5 ml for 4 hr at ambient temperature. The resin was removed by filtration and the filtrate added to 400 ml of diethyl ether and stirred for ½ hr. The resulting solid was isolated by centrifugation, washed well with ether and dried under reduced pressure to give 0.48 g of a white solid.

The crude material was injected in three equal aliquots onto two Vydac C-18 (25 x 250 mm, 10µ)columns in tandem and eluted using a 20 - 100% (80% acetonitrile/0.1% trifluoroacetic acid in water) over 120 min at a flow rate of 5 mL/min. Yields of the peptides were typically between 15-18 mg. The observed and calculated molecular weights were in good agreement.

### Example 2

### Preparation of mono- or di-substituted alpha-MSH derivative-Fc conjugates

### Deglycosylation of 7E3 IgG Fc

7E3 IgG Fc is a Fc portion of an IgG prepared by papain digestion to generate abciximab (CAS registry number 143653-53-6) using standard methods. 135 ml of Fc (5 mg/ml) was dialyzed into 10 mM Tris, pH 7.5. 100µl of PNGase F (500,000 u/ml) was added to the dialysate and the resulting solution incubated at 37° for 3 days. The deglycosylated Fc was purified on a TosoHaas phenyl 5PW column (5.5 x 200 mm, 10µ) eluted with the gradient of 0-50% buffer B at a flow rate of 11 ml/min (Buffer A: 0.1 M sodium phosphate, 1 M ammonium sulfate, pH 6.5; Buffer B: 0.1 M sodium phosphate, pH 6.5). Molecular Weight: Calcd: 49,864.4. Found: 49,868.4.

### Oxidation of deglycosylated Fc

55 ml of deglycosylated Fc (8.9 mg/ml) was dialyzed into 1% NaHCO₃, pH 8.4, to give 56.3 ml of 8.6 mg/ml. The concentration was adjusted to 5.1 mg/ml (10⁻⁴ mmol of protein/ml, equivalent to 2 x 10⁻⁴ mmol of N-terminal threonine/ml) by the addition of 40.6 ml of 1% NaHCO₃, pH 8.4. 11.9 ml of 12.5 mg/ml of methionine in 1% NaHCO₃, pH 8.4 was was added to the Fc solution. 2.12 ml (42.4 mg)of 20 mg/ml NaIO₄ in water was added to the Fc. The reaction mixture was gently agitated at ambient temperature for 15 minutes. Ethylene glycol (2.8 g, 2.3 ml) was added and the reaction gently agitated for an additional 20 minutes. The solution was dialyzed into 0.1 M NaOAc, pH 4.5 to give 120 ml of 4.0 mg/ml. The solution was divided into 2.5 ml aliquots, frozen at -20°C and used without further purification.

### Preparation of mono- and di-substituted alpha-MSH derivative-Fc conjugates

0.5 - 10 mg of the peptides having Formulae II-XV were added into the Fc aldehyde (typically 3.0 ml, 2.68 mg/ml). The tube was stored at 4° C overnight. 100 µL of 10 mg/ml of NaBH₃CN in water was added to the reaction mixture and the reaction was stored at 4° C overnight. Following the addition of 100-200 mg of ammonium sulfate, the sample was injected onto a TosoHaas phenyl or ether 5PW column (21.5 x 150 mm, 10µ) and eluted with the 0 - 100% gradient (Buffer A = 0.1 M sodium phosphate/1M ammonium sulfate, pH 6.5; Bufer B = 0.1 M sodium phosphate/1M ammonium sulfate, pH 6.5) over 180 minutes, collecting 6 ml fractions.

Column fractions were analyzed with an Agilent Bioanalyzer 2100 using a Protein 80 Kit and processed according to the manufacturer's instructions. Fractions containing the desired product were pooled, concentrated using an Amicon Ultra-15 Centrifugal Filter Device with a 10 kDa molecular weight cut-off and dialyzed into PBS using a Pierce Slide-a-Lyzer dialysis cassette with a molecular weight cut-off of 10 kDa. Typical yields were 1.2 - 12 mg. The observed and calculated molecular weights were in good accordance. Table 3 shows the prepared alpha-MSH derivative-Fc conjugates.

**Table 3.**

| Peptide Formula | Fc conjugate substitution | EC50 (nM) |
|---|---|---|
| II | mono | 12.04 |
| | di | 1.83 |
| III | di | 13.24 |
| IV | mono | 8.54 |
| | di | 6.21 |
| V | mono | |
| | di | |
| V | di | 14.40 |
| VII | di | 5.70 |
| VIII | mono | 53.61 |
| IX | mono | 35.65 |
| X | di | 1.39 |
| XI | mono | 4.41 |
| | di | 0.45 |
| XII | mono | 12.00 |
| | di | 1.83 |
| XIII | mono | 13.20 |
| | di | 13.20 |
| XIV | mono | |
| | di | 4.15 |
| XV | mono | 85.40 |
| | di | 6.30 |

### Example 3

### Biological activity of mono- or di-substituted alpha-MSH derivative-Fc conjugates

### Cyclic AMP (cAMP) Assay

For the assay, CHO cells at density 65,000 cells per well were plated onto 96-well tissue culture treated plates 100 µL per well in DMEM/F12, 10% FBS, 1% Sodium Pyruvate, 1% L-Glutamine, and the plates were incubated overnight at 37°C. Next day, the media was aspirated off cell culture plates, replaced with 90 µL of warmed serum-free DMEM/F12 plus 1mM IBMX (Sigma 17018) and incubated for 15 minutes at 37° C. 10 µL of serial dilutions (40 µM - 4 nM) of each alpha-MSH derivative-Fc conjugate diluted in 0.5% BSA in PBS were added to the 90 µL of IBMX media and cells and stimulated for 15 minutes at 37° C. The stimulation media was aspirated off and replaced with 100 µL of lysis buffer provided in the kit (Tropix cAMP-Screen™ System Chemiluminescent Immunoassay System from Applera-Applied Biosystems) and incubated for 30 minutes at 37° C. Cell lysate or cAMP standard (60 µl/well) was added to the assay plate. The concentrations of cAMP standard were from 0.006 to 6000 pmol cAMP per 60 µl. The cAMP-AP Conjugate was diluted 1:100 with Conjugate Dilution Buffer and 30 µl was added to each well of the provided anti-mouse antibody-coated plate in the Tropix kit followed by the addition of 60ul/well of anti-cAMP antibody. The plates were incubated at ambient temperature for 60 minutes with gentle shaking and then washed plate 6 times (300 µl/well) with provided wash buffer. 100 µl/well of substrate/enhancer solution was added and the plate covered with foil, and incubated 30 minutes at ambient temperature with gentle shaking. The plates were read on a Victor³V 1420 Multilabel Counter (Perkin Elmer) using luminescence measurements.

EC₅₀ values for the tested alpha-MSH derivative-Fc conjugates are shown in Table 3. Acylated alpha-MSH (Phoenix Pharamceuticals), (Ac-NH-Ser-Tyr-Ser-Met-Glu-His-Phe-Arg-Trp-Gly-Lys-Pro-Val-CO-NH₂) was used as a reference. The reference exhibited a sigmoidal dose-response curve with an EC₅₀ value of 0.1549 nM.

The alpha-MSH derivative-Fc conjugates mono-substituted Formula V-Fc, di-substituted Formula V-Fc, and mono-substituted Formula XIV-Fc conjugates were tested at a single dose of 0.26 µM in the cAMP assays. The conjugates stimulated cAMP levels an average of 35.663 pmol/well, 12.879 pmol/well, and 48.717 pmol/well, respectively.

### Example 4

### Selectivity of mono- or di-substituted alpha-MSH derivative-Fc conjugates

Receptor selectivity of onjugates formula X-Fc and formula VII-Fc were assayed using GeneBLAzer assay kits and CHO-K1 cell lines overexpressing MC1R (SEQ ID NO: 5), MC3R (SEQ ID NO: 6), MC4R (SEQ ID NO: 7), or MC5R (SEQ ID NO: 8) (Invitrogen, Carlsbad, CA) according to manufacturer's instruction. Incrase in the cAMP upon receptor activation was assessed using a beta-lactamase reporter gene under control of the Cyclic AMP Response Element (CRE). EC50 values for the assays are shown in Table 4.

**Table 4.**

| Conjugate | EC₅₀ (nM) | | | |
|---|---|---|---|---|
| | MC1R | MC3R | MC4R | MC5R |
| Formula VII-Fc | 48.4 | 2.9 | 2.4 | 9.6 |
| Formula X-Fc | 3.9 | 30.9 | 34 | 968.7 |

## Claims

1. An alpha-MSH derivative-Fc conjugate, wherein the alpha-MSH derivative has a structure shown in Formula X: 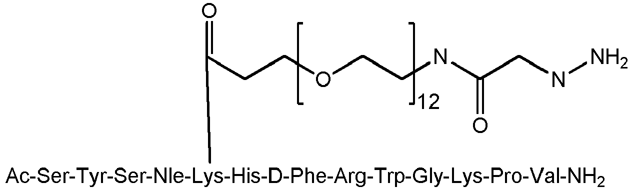 or Formula XI: 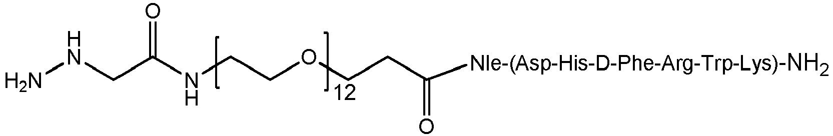 and the alpha-MSH derivative is conjugated to at least one Fc portion.

2. The alpha-MSH derivative-Fc conjugate of claim 1, wherein:
a) the alpha-MSH derivative is conjugated to the Fc via a linker; and/or
b) the Fc portion includes at least a portion of a hinge region, CH2 and CH3 domains; and/or
c) the alpha-MSH derivative is conjugated to the Fc via a linker and the Fc portion includes at least a portion of a hinge region, CH2 and CH3 domains, and wherein the alpha-MSH derivative-Fc conjugate may include one or more naturally occurring amino acids between the linker and the hinge.

3. The alpha-MSH derivative-Fc conjugate of any one of the preceding claims, wherein the alpha-MSH derivative is modified by at least one ethylene glycol unit.

4. The alpha-MSH derivative-Fc conjugate of any one of the preceding claims, wherein the alpha-MSH derivative is modified by 12 ethyleneoxy units.

5. The alpha-MSH derivative-Fc conjugate of claim 4, wherein the ethyleneoxy units are attached to an N-terminus of the alpha-MSH derivative or the ethyleneoxy units are attached to an internal lysine amino acid residue of the alpha-MSH derivative.

6. The alpha-MSH derivative-Fc conjugate of claim 2, wherein the linker is a hydrazine or a carbohydrazide group.

7. A pharmaceutical composition comprising the alpha-MSH derivative-Fc conjugate of any one of the preceding claims in combination with a pharmaceutically acceptable carrier.

8. A method of modulating a biological activity of a melanocortin receptor, comprising contacting the pharmaceutical composition of claim 7 with the melanocortin receptor.

9. The method of claim 8 wherein the melanocortin receptor is a melanocortin 1 receptor (MC1R), a melanocortin 3 receptor (MC3R), a melanocortin 4 receptor (MC4R), or a melanocortin 5 receptor (MC5R).

10. The alpha-MSH derivative-Fc conjugate of any one of claims 1-6 for use in treating a disease or condition.

11. The alpha-MSH derivative-Fc conjugate for use according to claim 10, wherein said disease or condition is obesity, type II diabetes, male erectile dysfunction, female sexual dysfunction, an inflammatory condition or fibrosis.

12. The pharmaceutical composition of claim 7 for use in treating a disease or condition.

13. The pharmaceutical composition for use according to claim 12, wherein the disease or condition is obesity or type II diabetes.

14. The pharmaceutical composition for use according to claim 12, wherein the disease or condition is male erectile dysfunction, female sexual dysfunction, an inflammatory condition or fibrosis.

15. The pharmaceutical composition for use according to claim 14 or the alpha-MSH derivative-Fc conjugate for use according to claim 11, wherein the inflammatory condition is allergic inflammation, gouty arthritis, rheumatoid arthritis, inflammatory bowel disease, post-ischemia renal injury, liver inflammation, ischemic brain damage or peripheral neuropathies.

## Patentansprüche

1. Alpha-MSH-Derivat-Fc-Konjugat, wobei das alpha-MSH-Derivat eine in Formel X: 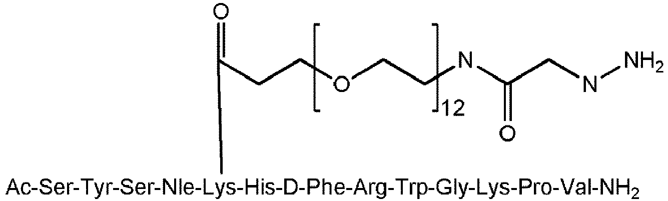 oder Formel XI: 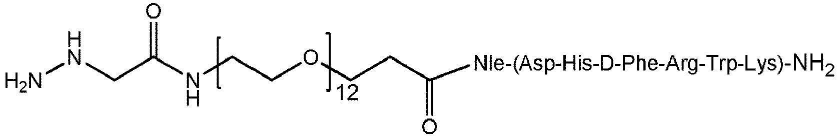 gezeigte Struktur aufweist und das alpha-MSH-Derivat an mindestens einen Fc-Teil gebunden ist.

2. Alpha-MSH-Derivat-Fc-Konjugat nach Anspruch 1, wobei:
a) das alpha-MSH-Derivat über einen Linker an das Fc konjugiert ist und/oder
b) der Fc-Teil mindestens einen Teil eines Scharnierbereichs, CH2- und CH3-Domänen enthält und/oder
c) das alpha-MSH-Derivat über einen Linker an das Fc gebunden ist und der Fc-Teil mindestens einen Teil eines Scharnierbereichs, CH2- und CH3-Domänen enthält und wobei das alpha-MSH-Derivat-Fc-Konjugat eine oder mehrere natürlich vorkommende Aminosäuren zwischen dem Linker und dem Scharnier enthalten kann.

3. Alpha-MSH-Derivat-Fc-Konjugat nach einem der vorhergehenden Ansprüche, wobei das alpha-MSH-Derivat durch mindestens eine Ethylenglykol-Einheit modifiziert ist.

4. Alpha-MSH-Derivat-Fc-Konjugat nach einem der vorhergehenden Ansprüche, wobei das alpha-MSH-Derivat durch 12 Ethylenoxy-Einheiten modifiziert ist.

5. Alpha-MSH-Derivat-Fc-Konjugat nach Anspruch 4, wobei die Ethylenoxy-Einheiten an einen N-Terminus des alpha-MSH-Derivats gebunden sind oder die Ethylenoxy-Einheiten an einen internen Lysin-Aminosäurerest des alpha-MSH-Derivats gebunden sind.

6. Alpha-MSH-Derivat-Fc-Konjugat nach Anspruch 2, wobei es sich bei dem Linker um eine Hydrazin- oder eine Carbohydrazidgruppe handelt.

7. Pharmazeutische Zusammensetzung, umfassend das alpha-MSH-Derivat-Fc-Konjugat nach einem der vorhergehenden Ansprüche in Kombination mit einem pharmazeutisch unbedenklichen Träger.

8. Verfahren zur Modulierung einer biologischen Aktivität eines Melanocortin-Rezeptors, bei dem man die pharmazeutische Zusammensetzung nach Anspruch 7 mit dem Melanocortin-Rezeptor in Berührung bringt.

9. Verfahren nach Anspruch 8, bei dem es sich bei dem Melanocortinrezeptor um einen Melanocortin-1-Rezeptor (MC1R), einen Melanocortin-3-Rezeptor (MC3R), einen Melanocortin-4-Rezeptor (MC4R) oder einen einen Melanocortin-5-Rezeptor (MC5R) handelt.

10. Alpha-MSH-Derivat-Fc-Konjugat nach einem der Ansprüche 1-6 zur Verwendung bei der Behandlung einer Erkrankung oder eines Leidens.

11. Alpha-MSH-Derivat-Fc-Konjugat zur Verwendung nach Anspruch 10, wobei es sich bei der Erkrankung oder dem Leiden um Obesitas, Typ-II-Diabetes, männliche erektile Dysfunktion, weibliche sexuelle Dysfunktion, ein entzündliches Leiden oder Fibrose handelt.

12. Pharmazeutische Zusammensetzung nach Anspruch 7 zur Verwendung bei der Behandlung einer Erkrankung oder eines Leidens.

13. Pharmazeutische Zusammensetzung zur Verwendung nach Anspruch 12, wobei es sich bei der Erkrankung oder dem Leiden um Obesitas oder Typ-II-Diabetes handelt.

14. Pharmazeutische Zusammensetzung zur Verwendung nach Anspruch 12, wobei es sich bei der Erkrankung oder dem Leiden um männliche erektile Dysfunktion, weibliche sexuelle Dysfunktion, ein entzündliches Leiden oder Fibrose handelt.

15. Pharmazeutische Zusammensetzung zur Verwendung nach Anspruch 14 oder alpha-MSH-Derivat-Fc-Konjugat zur Verwendung nach Anspruch 11, wobei es sich bei dem entzündlichen Leiden um allergische Entzündung, Gichtarthritis, rheumatoide Arthritis, entzündliche Darmerkrankung, Nierenverletzung nach Ischämie, Leberentzündung, ischämischen Hirnschaden oder periphere Neuropathien handelt.

## Revendications

1. Conjugué Fc-dérivé de l'α-MSH, le dérivé de l'α-MSH ayant une structure représentée à la formule X : 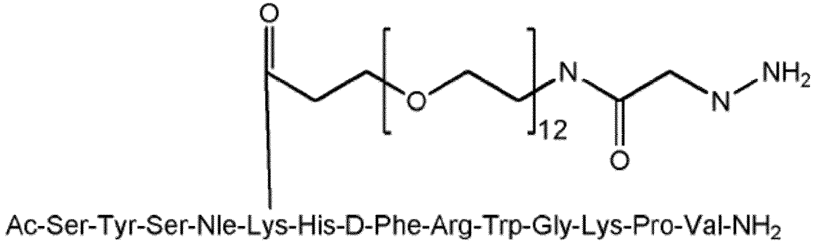 ou à la formule XI : 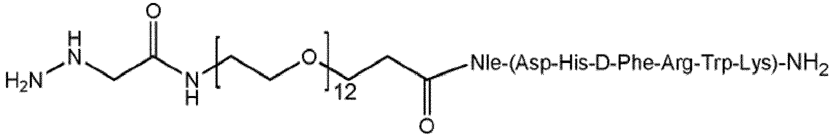 et le dérivé de l'α-MSH étant conjugué à au moins une portion Fc.

2. Conjugué Fc-dérivé de l'α-MSH selon la revendication 1, dans lequel :
a) le dérivé de l'α-MSH est conjugué à Fc via un coupleur ; et/ou
b) la portion Fc inclut au moins une portion d'une région charnière, des domaines CH2 et CH3 ; et/ou
c) le dérivé de l'α-MSH est conjugué à Fc via un coupleur et la portion Fc inclut au moins une portion d'une région charnière, des domaines CH2 et CH3, et le conjugué Fc-dérivé de l'α-MSH peut inclure au moins un acide aminé naturel entre le coupleur et la charnière.

3. Conjugué Fc-dérivé de l'α-MSH selon l'une quelconque des revendications précédentes, dans lequel le dérivé de l'α-MSH est modifié par au moins une unité éthylène glycol.

4. Conjugué Fc-dérivé de l'α-MSH selon l'une quelconque des revendications précédentes, dans lequel le dérivé de l'α-MSH est modifié par 12 unités éthylène glycol.

5. Conjugué Fc-dérivé de l'α-MSH selon la revendication 4, dans lequel les unités éthylène glycol sont attachées à une extrémité N du dérivé de l'α-MSH ou les unités éthylène glycol sont attachées à un résidu acide aminé lysine interne du dérivé de l'α-MSH.

6. Conjugué Fc-dérivé de l'α-MSH selon la revendication 2, dans lequel le coupleur est une hydrazine ou un groupe carbohydrazide.

7. Composition pharmaceutique contenant le conjugué Fc-dérivé de l'α-MSH selon l'une quelconque des revendications précédentes en association avec un vecteur pharmaceutiquement acceptable.

8. Procédé de modulation de l'activité biologique d'un récepteur de la mélanocortine, comprenant la mise en contact de la composition pharmaceutique selon la revendication 7 avec le récepteur de la mélanocortine.

9. Procédé selon la revendication 8, dans lequel le récepteur de la mélanocortine est un récepteur de la mélanocortine 1 (MC1R), un récepteur de la mélanocortine 3 (MC3R), un récepteur de la mélanocortine 4 (MC4R) ou un récepteur de la mélanocortine 5 (MC5R).

10. Conjugué Fc-dérivé de l'α-MSH selon l'une quelconque des revendications 1 à 6, destiné à être utilisé dans le traitement d'une maladie ou d'un état.

11. Conjugué Fc-dérivé de l'α-MSH destiné à être utilisé selon la revendication 10, dans lequel ladite maladie ou ledit état est l'obésité, le diabète de type II, la dysfonction érectile masculine, la dysfonction sexuelle féminine, un état inflammatoire ou une fibrose.

12. Composition pharmaceutique selon la revendication 7, destinée à être utilisée dans le traitement d'une maladie ou d'un état.

13. Composition pharmaceutique destinée à être utilisée selon la revendication 12, dans lequel ladite maladie ou ledit état est l'obésité ou le diabète de type II.

14. Composition pharmaceutique destinée à être utilisée selon la revendication 12, dans lequel ladite maladie ou ledit état est la dysfonction érectile masculine, la dysfonction sexuelle féminine, un état inflammatoire ou une fibrose.

15. Composition pharmaceutique destinée à être utilisée selon la revendication 14 ou conjugué Fc-dérivé de l'a-MSH destiné à être utilisé selon la revendication 11, la condition inflammatoire étant une inflammation allergique, une arthrite causée par la goutte, une arthrite rhumatoïde, une maladie inflammatoire chronique de l'intestin, une blessure rénale post-ischémique, une inflammation hépatique, un dommage cérébral ischémique ou des neuropathies périphériques.


### SEQUENCE LISTING

<110> Centocor Ortho Biotech Inc.
<120> MELANOCORTIN RECEPTOR BINDING CONJUGATES
<130> CEN5257USNP
<140> To Be Assigned
   <141> 2010-04-27
<150> 61175921
   <151> 2009-05-06
<150> 61176294
   <151> 2009-05-07
<160> 9
<170> FastSEQ for Windows Version 4.0
<210> 1
   <211> 13
   <212> PRT
   <213> Homo sapiens
<400> 1
   Ser Tyr Ser Met Glu His Phe Arg Trp Gly Lys Pro Val
    1               5                   10
<210> 2
   <211> 330
   <212> PRT
   <213> Homo sapiens
<400> 2
<210> 3
   <211> 330
   <212> PRT
   <213> Homo sapiens
<400> 3
<210> 4
   <211> 9
   <212> PRT
   <213> Artififial sequence
<220>
   <223> Portion of a human IgG variable region
<400> 4
   Gly Thr Leu Val Thr Leu Val Ser Ser
    1               5
<210> 5
   <211> 317
   <212> PRT
   <213> Homo sapiens
<400> 5
   Met Ala Val Gln Gly Ser Gln Arg Arg Leu Leu Gly Ser Leu Asn Ser
    1               5                  10                  15
   Thr Pro Thr Ala Ile Pro Gln Leu Gly Leu Ala Ala Asn Gln Thr Gly
               20                  25                  30
   Ala Arg Cys Leu Glu Val Ser Ile Ser Asp Gly Leu Phe Leu Ser Leu
           35                  40                  45
   Gly Leu Val Ser Leu Val Glu Asn Ala Leu Val Val Ala Thr Ile Ala
       50                  55                  60
   Lys Asn Arg Asn Leu His Ser Pro Met Tyr Cys Phe Ile Cys Cys Leu
   65                  70                  75                  80
   Ala Leu Ser Asp Leu Leu Val Ser Gly Ser Asn Val Leu Glu Thr Ala
                   85                  90                  95
   Val Ile Leu Leu Leu Glu Ala Gly Ala Leu Val Ala Arg Ala Ala Val
               100                 105                 110
   Leu Gln Gln Leu Asp Asn Val Ile Asp Val Ile Thr Cys Ser Ser Met
           115                 120                 125
   Leu Ser Ser Leu Cys Phe Leu Gly Ala Ile Ala Val Asp Arg Tyr Ile
       130                 135                 140
   Ser Ile Phe Tyr Ala Leu Arg Tyr His Ser Ile Val Thr Leu Pro Arg
   145                 150                 155                 160
   Ala Arg Arg Ala Val Ala Ala Ile Trp Val Ala Ser Val Val Phe Ser
                   165                 170                 175
   Thr Leu Phe Ile Ala Tyr Tyr Asp His Val Ala Val Leu Leu Cys Leu
               180                 185                 190
   Val Val Phe Phe Leu Ala Met Leu Val Leu Met Ala Val Leu Tyr Val
           195                 200                 205
   His Met Leu Ala Arg Ala Cys Gln His Ala Gln Gly Ile Ala Arg Leu
       210                 215                 220
   His Lys Arg Gln Arg Pro Val His Gln Gly Phe Gly Leu Lys Gly Ala
   225                 230                 235                 240
   Val Thr Leu Thr Ile Leu Leu Gly Ile Phe Phe Leu Cys Trp Gly Pro
                   245                 250                 255
   Phe Phe Leu His Leu Thr Leu Ile Val Leu Cys Pro Glu His Pro Thr
               260                 265                 270
   Cys Gly Cys Ile Phe Lys Asn Phe Asn Leu Phe Leu Ala Leu Ile Ile
           275                 280                 285
   Cys Asn Ala Ile Ile Asp Pro Leu Ile Tyr Ala Phe His Ser Gln Glu
       290                 295                 300
   Leu Arg Arg Thr Leu Lys Glu Val Leu Thr Cys Ser Trp
   305                 310                 315
<210> 6
   <211> 323
   <212> PRT
   <213> Homo sapiens
<400> 6
   Met Asn Ala Ser Cys Cys Leu Pro Ser Val Gln Pro Thr Leu Pro Asn
    1               5                  10                  15
   Gly Ser Glu His Leu Gln Ala Pro Phe Phe Ser Asn Gln Ser Ser Ser
               20                  25                  30
   Ala Phe Cys Glu Gln Val Phe Ile Lys Pro Glu Val Phe Leu Ser Leu
           35                  40                  45
   Gly Ile Val Ser Leu Leu Glu Asn Ile Leu Val Ile Leu Ala Val Val
       50                  55                  60
   Arg Asn Gly Asn Leu His Ser Pro Met Tyr Phe Phe Leu Cys Ser Leu
   65                  70                  75                  80
   Ala Val Ala Asp Met Leu Val Ser Val Ser Asn Ala Leu Glu Thr Ile
                   85                  90                  95
   Met Ile Ala Ile Val His Ser Asp Tyr Leu Thr Phe Glu Asp Gln Phe
               100                 105                 110
   Ile Gln His Met Asp Asn Ile Phe Asp Ser Met Ile Cys Ile Ser Leu
           115                 120                 125
   Val Ala Ser Ile Cys Asn Leu Leu Ala Ile Ala Val Asp Arg Tyr Val
       130                 135                 140
   Thr Ile Phe Tyr Ala Leu Arg Tyr His Ser Ile Met Thr Val Arg Lys
   145                 150                 155                 160
   Ala Leu Thr Leu Ile Val Ala Ile Trp Val Cys Cys Gly Val Cys Gly
                   165                 170                 175
   Val Val Phe Ile Val Tyr Ser Glu Ser Lys Met Val Ile Val Cys Leu
               180                 185                 190
   Ile Thr Met Phe Phe Ala Met Met Leu Leu Met Gly Thr Leu Tyr Val
           195                 200                 205
   His Met Phe Leu Phe Ala Arg Leu His Val Lys Arg Ile Ala Ala Leu
       210                 215                 220
   Pro Pro Ala Asp Gly Val Ala Pro Gln Gln His Ser Cys Met Lys Gly
   225                 230                 235                 240
   Ala Val Thr Ile Thr Ile Leu Leu Gly Val Phe Ile Phe Cys Trp Ala
                   245                 250                 255
   Pro Phe Phe Leu His Leu Val Leu Ile Ile Thr Cys Pro Thr Asn Pro
               260                 265                 270
   Tyr Cys Ile Cys Tyr Thr Ala His Phe Asn Thr Tyr Leu Val Leu Ile
           275                 280                 285
   Met Cys Asn Ser Val Ile Asp Pro Leu Ile Tyr Ala Phe Arg Ser Leu
       290                 295                 300
   Glu Leu Arg Asn Thr Phe Arg Glu Ile Leu Cys Gly Cys Asn Gly Met
   305                 310                 315                 320
   Asn Leu Gly
<210> 7
   <211> 332
   <212> PRT
   <213> Homo sapiens
<400> 7
<210> 8
   <211> 325
   <212> PRT
   <213> Homo sapiens
<400> 8
   Met Asn Ser Ser Phe His Leu His Phe Leu Asp Leu Asn Leu Asn Ala
    1               5                  10                  15
   Thr Glu Gly Asn Leu Ser Gly Pro Asn Val Lys Asn Lys Ser Ser Pro
               20                  25                  30
   Cys Glu Asp Met Gly Ile Ala Val Glu Val Phe Leu Thr Leu Gly Val
           35                  40                  45
   Ile Ser Leu Leu Glu Asn Ile Leu Val Ile Gly Ala Ile Val Lys Asn
       50                  55                  60
   Lys Asn Leu His Ser Pro Met Tyr Phe Phe Val Cys Ser Leu Ala Val
   65                  70                  75                  80
   Ala Asp Met Leu Val Ser Met Ser Ser Ala Trp Glu Thr Ile Thr Ile
                   85                  90                  95
   Tyr Leu Leu Asn Asn Lys His Leu Val Ile Ala Asp Ala Phe Val Arg
               100                 105                 110
   His Ile Asp Asn Val Phe Asp Ser Met Ile Cys Ile Ser Val Val Ala
           115                 120                 125
   Ser Met Cys Ser Leu Leu Ala Ile Ala Val Asp Arg Tyr Val Thr Ile
       130                 135                 140
   Phe Tyr Ala Leu Arg Tyr His His Ile Met Thr Ala Arg Arg Ser Gly
   145                 150                 155                 160
   Ala Ile Ile Ala Gly Ile Trp Ala Phe Cys Thr Gly Cys Gly Ile Val
                   165                 170                 175
   Phe Ile Leu Tyr Ser Glu Ser Thr Tyr Val Ile Leu Cys Leu Ile Ser
               180                 185                 190
   Met Phe Phe Ala Met Leu Phe Leu Leu Val Ser Leu Tyr Ile His Met
           195                 200                 205
   Phe Leu Leu Ala Arg Thr His Val Lys Arg Ile Ala Ala Leu Pro Gly
       210                 215                 220
   Ala Ser Ser Ala Arg Gln Arg Thr Ser Met Gln Gly Ala Val Thr Val
   225                 230                 235                 240
   Thr Met Leu Leu Gly Val Phe Thr Val Cys Trp Ala Pro Phe Phe Leu
                   245                 250                 255
   His Leu Thr Leu Met Leu Ser Cys Pro Gln Asn Leu Tyr Cys Ser Arg
               260                 265                 270
   Phe Met Ser His Phe Asn Met Tyr Leu Ile Leu Ile Met Cys Asn Ser
           275                 280                 285
   Val Met Asp Pro Leu Ile Tyr Ala Phe Arg Ser Gln Glu Met Arg Lys
       290                 295                 300
   Thr Phe Lys Glu Ile Ile Cys Cys Arg Gly Phe Arg Ile Ala Cys Ser
   305                 310                 315                 320
   Phe Pro Arg Arg Asp
                   325
<210> 9
   <211> 297
   <212> PRT
   <213> Homo sapiens
<400> 9